# EUROPÄISCHE PATENTANMELDUNG

(11) **EP 4 648 058 A1**
(43) Veröffentlichungstag der Anmeldung: **12.11.2025**
(21) Anmeldenummer: 24174354.1
(22) Anmeldetag: 06.05.2024
(51) Int. Cl.: G16H 40/20, A61M 1/14, G16H 40/63, G06F 21/62

(54) **BEDIENERLEVEL UND ROLLENSPEZIFISCHE AUTORISATION ZUR BEDIENUNG EINER INFUSIONSPUMPE**

(71) Anmelder: B. Braun Melsungen AG, 34212 Melsungen (DE)
(72) Erfinder: BUERGER, Maria, 34323 Malsfeld (DE); HOLZ, Stefan, 36199 Rotenburg (DE)
(74) Vertreter: Winter, Brandl - Partnerschaft mbB

(57) **Zusammenfassung**

Die vorliegende Offenbarung betrifft ein Sicherungssystem (1) eines oder für ein medizinisches Gerät (2) zur Erteilung von identitätsspezifisch auswählbaren Berechtigungsfreigaben an Bedienpersonen für das Ausführen von berechtigungsabhängigen Manipulationen am medizinischen Gerät (2), mit einem Datenspeicher (4), der Daten enthält, die eine Anzahl von authentifizierbaren Bedienpersonen ausweisen, denen individuelle Berechtigungsstufen zugeordnet oder zuordenbar sind, einer Authentisierungsvorrichtung (6), die dazu vorgesehen und ausgebildet ist, die Identität einer aktuellen Bedienperson festzustellen und einer Authentifizierungsvorrichtung (8), die dazu vorgesehen und ausgebildet ist, die Identität der authentisierten Bedienperson anhand der im Datenspeicher (4) abgespeicherten Daten zu bestätigen, wobei eine Berechtigungsfreigabe-Auswahlvorrichtung (10), die dafür vorgesehen und ausgebildet ist, bei einer erfolgreichen Authentifizierung eine vorbestimmte Berechtigungsfreigabe in Abhängigkeit der Berechtigungsstufe der authentifizierten Bedienperson auszuwählen und zu erteilen. Ferner betrifft die vorliegende Offenbarung ein Verfahren zur Erteilung von identitätsspezifischen Berechtigungsfreigaben an Bedienpersonen für das Ausführen von berechtigungsabhängigen Manipulationen an einem medizinischen Gerät (2).

## Beschreibung

### Technisches Gebiet

Die vorliegende Offenbarung betrifft ein Sicherungssystem, insbesondere elektronisches Zugangs-Sicherungssystem eines oder für ein medizinisches Gerät (Infusionspumpe, Blutbehandlungsmaschine und/oder dergleichen intensivmedizinische Einrichtungen) sowie ein (automatisiertes) Verfahren zur Erteilung einer Berechtigungsfreigabe an einen bestimmten Nutzer/Nutzertypen für ein bestimmtes medizinisches Gerät/Gerätetyp.

### Hintergrund der Offenbarung

Um an einem Gerät sensible (kritische) Manipulationen/Einstellungen vornehmen zu können, ist in der Regel ein Berechtigungsnachweis erforderlich, der in einer geeigneten Weise wie nachfolgend beispielhaft beschrieben erbracht werden muss.

Als Authentifizierung bezeichnet man hierfür allgemein zunächst die Prüfung eines Identitätsnachweises auf seine Authentizität. Im Beispiel eines Betriebssystems eines sensiblen Geräts, welches Zugang zu einem gesicherten Bereich, beispielsweise einer Einstellung / einer Manipulation des Geräts, gewähren kann, behauptet eine Bedienperson als erstes ihre Zugangsberechtigung, indem sie ein zuvor festgelegtes Authentisierungsmittel bzw. einen entsprechenden Identifikationsnachweis (Chipkarte) vorlegt/einliest oder einen Zugangs-Code eingibt. Das Betriebssystem identifiziert daraufhin die Bedienperson (Benutzer) oder den Bedienpersonentyp anhand des Authentisierungsmittels und führt anschließend die Authentifizierung durch, also die Verifizierung der erbrachten Behauptung über die Authentizität. Im Fall einer Code-Eingabe erfolgt dies nach dem Prinzip eines Zahlenschlosses, wohingegen im Fall eines Identifikationsnachweises zunächst dessen grundsätzliche Echtheit und anschließend dessen Übereinstimmung mit einem hinterlegten Muster erfolgt. Erst wenn diese Verifizierung erfolgreich ist, wird der Bedienperson die Zugangsberechtigung, üblicherweise für die Dauer einer Sitzung oder einer bestimmten Zeit, erteilt.

### Stand der Technik

Bei dem Einsatz von medizinischen Geräten ist es ebenfalls wichtig, dass gewisse Manipulationen (Funktionen / Aktivitäten bzw. Einstellungen) des medizinischen Gerätes geschützt sind. Dies ist notwendig, damit nicht autorisierte Bedienpersonen oder fremde Personen keinen Zugriff auf spezifische Manipulationen, unter anderem Einstellungen und Funktionen des medizinischen Geräts haben. Einer der Gründe für diese Sicherung ist, dass es bei Anwendungsfehlern oder mutwilligen Eingriffen zu einem Schaden an dem medizinischen Gerät und/oder einer Schädigung einer mit dem medizinischen Gerät in Therapie befindlichen Person kommen kann.

Bei solchen medizinischen Geräten, unter anderem bei Infusionspumpen oder auch Blutbehandlungsmaschinen, ist es aktuell bekannt, dass ein Schutz vor Manipulationen, beispielsweise von sicherheitsrelevanten Funktionen, wie z.B. die Bereitstellung oder Zufuhr kritischer Medikamente über eine Codesperre erfolgt. Der dafür verwendete Code ist in der Regel vom Hersteller in der Software des medizinischen Geräts hart kodiert, d.h. fest einprogrammiert. Aktuell erfolgt die Definition eines solchen Codes, ob beispielsweise eine Medikamentenzufuhr vor bestimmten Manipulationen geschützt ist, mithilfe von bestimmten Service Tools, wie beispielsweise einer Medikamenten- und/oder Therapie-Datenbank. In einem solche Service Tool sind eine Anzahl von kritischen Medikamenten (z.B. Schmerzmittel) und/oder eine Anzahl von kritischen Behandlungstherapien hinterlegt, welche den Einsatz einer Code-Sicherung des medizinischen Geräts bedürfen.

Wird demzufolge ein solches kritisches Medikament mittels des medizinischen Geräts verabreicht oder eine solche kritische Behandlungstherapie mittels des medizinischen Geräts durchgeführt, ist die Eingabe des fest einprogrammierten Freigabecodes notwendig, um bestimmte Einstellungen bei der laufenden Therapie am medizinischen Gerät durchführen zu können.

Üblicherweise haben verschiedene Bedienpersonengruppen (Nutzertypen) unterschiedlichen Zugriff auf die medizinischen Geräte, d.h. es existiert in der Regel eine Nutzergruppe, die ausschließlich grundsätzlich freie Manipulationen durchführen dürfen und eine solche Nutzergruppe, die auch Code-gesicherte Manipulationen freischalten können. In anderen Worten ausgedrückt sind im Fall von zu verabreichenden, unkritischen Medikamenten und/oder Therapien, Gerätemanipulationen häufig freigeschaltet, wohingegen bei kritischen Medikamenten und/oder Therapien die fest einprogrammierte Code-Sperre zum Einsatz kommt, sodass nur noch wenige ausgewählte Manipulationen grundsätzlich freigeschaltet bleiben und für andere Manipulationen die Code-Eingabe erforderlich ist.

Diese Sicherungssystematik ist jedoch nachteilig, da die Anzahl an Manipulationen durch die Hartkodierung des Codes festgelegt ist und eine Anpassung und/oder Erweiterung der Code-spezifischen Manipulationen somit eingeschränkt oder sogar unmöglich wird. In anderen Worten ausgedrückt besteht bei den aktuellen medizinischen Geräten lediglich die Möglichkeit, über den einprogrammierten Code sämtliche sensible (für die Patientensicherheit relevante) Funktionen/Manipulationen freizuschalten oder zu sperren.

### Kurzbeschreibung der Offenbarung

Der vorliegenden Offenbarung liegt die Aufgabe zugrunde, die zuvor beschriebenen Nachteile zu beseitigen oder zumindest zu verbessern. Ein bevorzugtes Ziel der vorliegenden Offenbarung ist es, ein medizinisches (Zugangs-) Sicherungssystem bereitzustellen, welches eine höhere Flexibilität mit Bezug auf die Vergabe einer Zugangsberechtigung bietet.

Diese Aufgabe wird gelöst durch ein Sicherungssystem mit den Merkmalen des Anspruchs 1 und einem Verfahren mit den Merkmalen des Anspruchs 8.

Der Kerngedanke der vorliegenden Offenbarung besteht darin, zunächst eine (erste) Datenbank zu etablieren oder bereitzustellen, in der eine Anzahl (Mehrzahl) von unterschiedlichen Bedienpersonentypen oder-gruppen (flexibel) eingegeben werden können oder eingegeben sind, also eine Mehrzahl von unterschiedlichen Hierarchiestufen hinterlegbar oder hinterlegt sind, wobei jeder Hierarchiestufe ein bestimmter oder bestimmbarer Autorisierungsgrad zugeordnet ist oder wird oder jede Hierarchiestufe einem bestimmten oder bestimmbaren Autorisierungsgrad entspricht. Hinter jedem Autorisierungsgrad steht eine Anzahl ausgewählter bzw. auswählbarer, (zumindest teilweise zueinander unterschiedlicher) Manipulationen oder Manipulationszusammenstellungen (Manipulationslinien). D.h. jedem Autorisierungsgrad ist (kann) eine vordefinierte, oder individuell (frei) zusammengestellte Auswahl an Manipulationen zugewiesen (werden), wobei sich die zugewiesenen Manipulationen unter den Autorisierungsgraden (d.h. von Autorisierungsgrad zu Autorisierungsgrad) zumindest teilweise unterscheiden können. Des Weiteren ist es vorgesehen, eine weitere (zweite) Datenbank zu etablieren oder bereitzustellen, in der eine Anzahl (Mehrzahl) von unterschiedlichen Medikamenten und/oder Therapien (flexibel) eingegeben werden können oder eingegeben sind, also eine Mehrzahl von unterschiedlichen Medikamenten und/oder Therapien hinterlegbar oder hinterlegt sind, denen jeweils eine oder mehrere für eine Freigabe vorgesehene Manipulationen oder ganze Autorisierungsgrade (umfassend die zugehörigen Manipulationen) zugeordnet oder zuordenbar sind.

Schließlich ist für die geräteseitige Bestätigung/Verifikation der Zugehörigkeit einer Bedienperson zu zumindest einer der bereits hinterlegten oder noch zu hinterlegenden Hierarchiestufen ein Hierarchiestufen-bezogener Nachweis beispielsweise in Form eines jeweiligen Identifikationsnachweises (Chipkarte, Ausweis, etc.) oder eines Codes gemäß dem eingangs genannten Stand der Technik seitens der Bedienperson zu erbringen, um die Freigabe für jene Manipulationen entsprechend dem jeweils Personen-zugeordneten Autorisierungsgrad (Hierarchiestufe) sowie entsprechend dem jeweiligen Medikament und/oder Therapie zu erhalten.

Die Offenbarung betrifft demzufolge ein (Zugangs-) Sicherungssystem eines oder für ein medizinisches Gerät, insbesondere eine Infusionspumpe, zur Bereitstellung/Zuteilung von Medikamenten- und/oder Therapie-spezifisch auswählbaren Berechtigungsfreigaben an Bedienpersonen oder Bedienpersonengruppen für das Ausführen von berechtigungsabhängigen Manipulationen am medizinischen Gerät. Das Sicherungssystem umfasst dabei einen ersten Datenspeicher oder Datensatz (geräteintern oder geräteextern), der Daten enthält oder in den Daten eingebbar sind, die eine Mehrzahl von Bedienpersonengruppen oder Hierarchiestufen ausweisen, denen zueinander unterschiedlichen Autorisierungsgrade zugeordnet oder zuordenbar sind, wobei jeder Autorisierungsgrad für bestimmte oder bestimmbare Manipulationen am medizinischen Gerät steht. Weiterhin umfasst das Sicherungssystem einen zweiten Datenspeicher oder Datensatz (geräteintern oder geräteextern), der Daten enthält oder in den Daten eingebbar sind, die eine Mehrzahl von unterschiedlichen Medikamenten und/oder Therapien ausweisen, denen jeweils bestimmte oder bestimmbare, freizuschaltende Manipulationen oder zumindest einer der zueinander unterschiedlichen Autorisierungsgrade gemäß der ersten Datenbank /dem ersten Datensatz zugeordnet oder zuordenbar ist. Schließlich umfasst das Sicherungssystem eine Berechtigungsfreigabe-Auswahlvorrichtung, die dafür vorgesehen und ausgebildet ist, einer Bedienperson in Abhängigkeit eines von dieser erbrachten Berechtigungsnachweises zumindest eine der Hierarchiestufen aus dem ersten Datenspeicher/Datensatz zuzuordnen und jene darin enthaltenen Manipulationen freizuschalten, welche mit den Medikamenten-/Therapie-bezogenen Manipulationen gemäß dem zweiten Datenspeicher/Datensatz übereinstimmen.

Zur Erbringung des Berechtigungsnachweises sind bevorzugt folgende Vorrichtungen vorgesehen:
- Eine Authentisierungsvorrichtung, die dafür ausgebildet ist, die Identität einer aktuellen Bedienperson festzustellen und
- eine Authentifizierungsvorrichtung, die dafür ausgebildet ist, die Identität der authentisierten Bedienperson anhand von im Datenspeicher abgespeicherten Daten zu bestätigen.

In anderen Worten gewährt das (medizinische) elektronische Sicherungssystem eine Manipulation von/an einem medizinischen Gerät. Hierfür ist bevorzugt eine Authentisierungsvorrichtung (beispielsweise ein Kartenleser, Scanner, Eingabefeld zur Eingabe eines Codes, etc.) vorgesehen, welche eine Bedienperson für seine Identifizierung beziehungsweise für den Nachweis seiner Identität nutzt. Die Bedienperson weist sozusagen ihre Bediendaten / Nutzerdaten / Identität an dieser Vorrichtung nach. Weiter vorzugsweise wird mithilfe einer Authentifizierungsvorrichtung dieser Nachweis bzw. die Identifizierung dann auf Richtigkeit geprüft. Die Berechtigungsfreigabe-Auswahlvorrichtung ist anschließend dazu eingerichtet, basierend auf den (vorab gespeicherten) Daten in den Datenspeichern zu entscheiden, welche Manipulationen bei dem jeweiligen aktuell zu verabreichenden Medikament oder der aktuell durchzuführenden Therapie an dem medizinischen Gerät für die jeweilige Bedienperson der entsprechenden Hierarchiestufe freigegeben werden und welche Manipulationen nicht freigegeben werden. In anderen Worten ausgedrückt hat das System Datenspeicher oder kann auf solche Datenspeicher zugreifen, in welchen Personen oder Personengruppen/Hierarchiestufen einschließlich Hierarchiestufenzugeordneter Manipulationen in einem (ersten) Datensatz und eine Anzahl an einzelnen Manipulationen oder eine Anzahl von unterschiedlichen Manipulationszusammenstellungen in einem anderen (zweiten) Datensatz abgespeichert sind, die in ausgewählter Weise verschiedenen Medikamenten und/oder Therapien zugeordnet sind, wobei die Berechtigungsfreigabe-Auswahlvorrichtung einer authentifizierten Person bestimmte Manipulationen oder eine Manipulationszusammenstellung entsprechend der dieser Person zugewiesenen Hierarchiestufe zuordnet. Je nachdem, welches Medikament aktuell verabreicht wird oder welche Therapie durchgeführt wird, reicht dann die, der betreffenden Person, zugewiesene Hierarchiestufe gemäß erstem Datensatz aus, die dieser Hierarchiestufe zugeordneten Manipulationen (gemäß erstem Datensatz) sämtlich freizugeben oder nur einige Manipulationen in dieser Hierarchiestufe (gemäß ersten Datensatz) freizugeben, welche mit den, dem aktuellen Medikament oder der aktuellen Therapie zugeordneten Manipulationen (gemäß zweitem Datensatz) übereinstimmen.

Vorteilhaft an der Offenbarung ist, dass identitätsspezifische Berechtigungsfreigaben erteilbar oder erteilt werden, wodurch voneinander verschiedenen Bedienpersonen verschiedene Berechtigungsfreigaben aufweisen, die wiederum unterschiedlichen Medikamenten und/oder Therapien zugeordnet sind. Auf diese Weise ist es möglich, flexibel Bedienpersonen einer bestimmten Hierarchiestufe für die damit verknüpften Manipulationen bei manchen Medikamenten und/oder Therapien teilweise oder ganz freizuschalten und bei anderen Medikamenten und/oder Therapien teilweise oder ganz zu sperren. Auch ist es möglich, diese Zuordnungen beliebig zu ändern oder durch weitere Hierarchiestufen und deren Zuordnungen zu Medikamenten und/oder Therapien zu erweitern.

Es hat sich gezeigt, dass durch die vorstehend beschriebene Offenbarung eine hohe Patientensicherheit bei gleichzeitig hoher Bedienfreundlichkeit erzielt werden kann.

Vorzugsweise ist das medizinische Gerät eine Infusionspumpe.

Bevorzugt kann ein einziger Datenspeicher / eine einzige Datenbank in den ersten und zweiten Datenspeicher unterteilt sein oder den ersten und zweiten Datensatz enthalten, wobei in dem ersten Datensatz die Hierarchiestufen/die Berechtigungsstufen mit den jeweils zugeordneten Manipulationen abgespeichert oder abspeicherbar sind und in dem zweiten Datensatz die Medikamente und/oder Therapien mit entsprechend zugeordneten freizugebenden Manipulationen oder ganzen Berechtigungsstufen abgespeichert oder abspeicherbar sind. Damit ist eine Abkopplung von den hart kodierten Eingaben des Herstellers im Softwarecode möglich. Insgesamt kann damit herstellunabhängig entschieden werden, welche Manipulationen identitätsspezifisch und Medikamenten-/Therapie-bezogen freigebbar sind.

Die beiden Datensätze können dabei an dem medizinischen Gerät programmiert sein oder werden, oder extern zum medizinischen Gerät an einer zum medizinischen Gerät separaten Vorrichtung, beispielsweise einem Computer, programmiert sein oder werden. Bei einer Programmierung an einem vom medizinischen Gerät separaten Computer ist es von Vorteil, wenn die Datensätze anschließend an den/die Datenspeicher übertragen werden. Diese Übertragung kann mithilfe einer Kabelverbindung erfolgen oder alternativ mittels einer Funkübertragung erfolgen, welche Bestandteil des Sicherungssystems sein können. Beispielhafte Funkübertragungen sind mittels WiFi, mittels Bluetooth oder mittels NFC möglich. Vorteilhaft an der Programmierung an einem externen Computer ist, dass eine dritte Person, welche mit der Festlegung der Hierarchie-/Berechtigungsstufen vertraut und beauftragt ist, diese Datensätze standortunabhängig vom medizinischen Gerät festlegen kann.

Vorzugsweise erfolgt nach einer erfolgreichen Authentifizierung ein Abgleich der dieser Person zugeordneten Hierarchie-/Berechtigungsstufe mit der abgespeicherten Hierarchie-/Berechtigungsstufe gemäß dem ersten Datensatz, um bei einer Übereinstimmung dieser beiden Stufen die Berechtigungsfreigabe zu erteilen. Sofern keine Übereinstimmung zwischen den jeweiligen Hierarchie-/Berechtigungsstufen gegeben ist, erfolgt keine Berechtigungsfreigabe und die Manipulation(en) ist/sind gesperrt.

Bevorzugt kann die Hierarchie-/Berechtigungsstufe der Bedienperson eine Variable X aufweisen. Dieser Wert X kann vorzugsweise einen von zwei voneinander verschiedenen Werten aufweisen. Beispielsweise ein Binärcode mit dem Wert "1" oder "0", oder ein Wahrheitswert mit dem Wert "wahr" oder "falsch". Alternativ sind auch alle anderen Werte zur Unterscheidung für eine zweistufige Abgrenzung denkbar. Weiter alternativ kann die Variable X eine n-verschiedene Anzahl an Werten aufweisen, so dass eine Stufung der Berechtigungsstufe gegeben ist. n kann dabei eine positive, endliche Zahl sein.

Bevorzugt kann die Hierarchie-/Berechtigungsstufe gemäß erstem Datensatz eine Variable Y aufweisen. Diese Variable Y kann vorzugsweise einen von zwei voneinander verschiedenen Werten aufweisen. Beispielsweise ein Binärcode mit dem Wert "1" oder "0", oder ein Wahrheitswert mit dem Wert "wahr" oder "falsch". Alternativ sind auch alle anderen Werte zur Unterscheidung zwischen einer zweistufigen Abgrenzung denkbar. Weiter alternativ kann die Variable Y eine n-verschiedene Anzahl an Werten aufweisen, so dass eine Stufung der Berechtigungsstufe gegeben ist. n kann dabei eine positive, endliche Zahl sein.

Von Vorteil ist, wenn nach einer erfolgreichen Authentifizierung ein Abgleich der Berechtigungsstufe der Bedienperson, vorliegend der Variable X, mit der Berechtigungsstufe gemäß dem ersten Datensatz, vorliegend der Variable Y, erfolgt, um bei einer Übereinstimmung dieser Stufen die Berechtigungsfreigabe zu erteilen. Mithilfe dieses Abgleichs ist eine eindeutige Berechtigungsfreigabe bzw. Autorisierung möglich, die gewährleistet, dass der Bedienperson nur für die ihr zugewiesene Manipulation Medikamenten-/Therapieabhängig eine Freigabe erhält.

Von Vorteil ist, wenn im ersten Datensatz einer Bedienperson (zusätzlich) eine Identitätskennung zugeordnet ist. Mithilfe der Identitätskennung ist die Bedienperson klar identifizierbar. Vorzugsweise ist die Identitätskennung ein numerischer oder alphanumerischer Code.

Bevorzugt kann die authentifizierbare Bedienperson einer Bediengruppe zugeordnet sein oder zuordenbar sein. Eine Bediengruppe weist den Vorteil auf, dass in dieser Gruppe eine Vielzahl von Bedienpersonen zuordenbar oder zugeordnet sind. Somit sind weniger einzelne Bedienpersonen mit jeweiligen Berechtigungsstufen zu definieren. Bei den einzelnen Bediengruppen kann es sich beispielhaft um das Pflegepersonal, den Arzt / die Ärztin an der Station, das Schmerzteam, die Stationsaufsicht usw. handeln. Die Bediengruppen können somit insgesamt verschiedene Hierarchiestufen abbilden. Grundliegend können diese einzelnen Bediengruppen verschiedene Qualifikationen abbilden. Weiterhin vorteilhaft an dieser Unterteilung ist, dass für jede diese Bediengruppen nur spezifischen Manipulationen definiert sind oder definierbar sind.

Der erste Datensatz und der zweite Datensatz können jeweils als eine Datenmatrix abgespeichert sein. Eine beispielhafte Ausgestaltung der Datenmatrix für den ersten Datensatz kann der nachfolgenden Tabelle 1 entnommen werden.

Beispiel eines Freigabekonzepts:

**Tabelle 1**

| **Freigabe-Matrix** | Hierarchiestufe | | | | |
|---|---|---|---|---|---|
| Geschützte Funktion: | Arzt/Arztin Station | SchmerzTeam | StationsAufsicht | Pflege**Personal** | (weitere...) |
| | Code: 8657 | Code: 5481 | Code: 3251 | Code: 1567 | |
| Flussrate ändern | X | X | | | |
| Einmalartikelwechsel | X | X | X | X | |

Die vorliegende Offenbarung ist jedoch nicht auf die vier beispielhaften Hierarchie-/Berechtigungsstufen und die zwei Manipulationen beschränkt. Insgesamt kann eine Vielzahl von Hierarchie-/Berechtigungsstufen und eine Vielzahl von Manipulationen definiert sein.

Eine beispielhafte Ausgestaltung der Datenmatrix für den zweiten Datensatz kann der nachfolgenden Tabelle 2 entnommen werden.

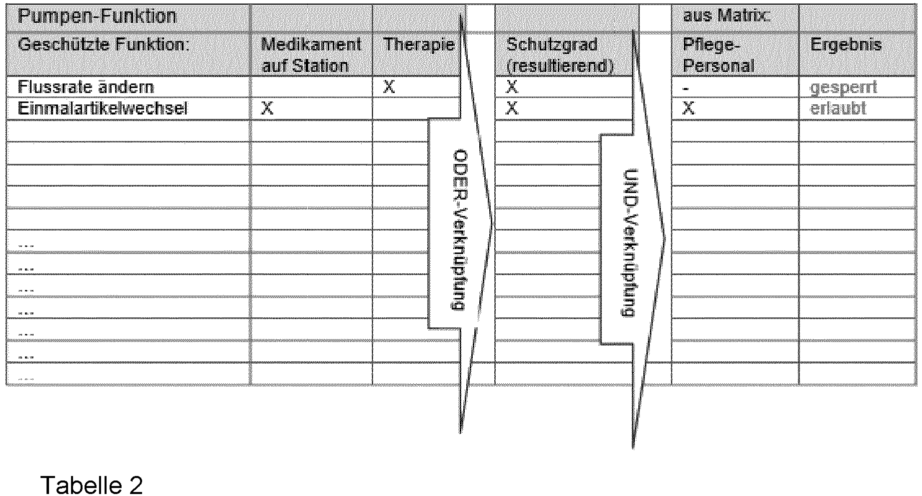

Die vorliegende Offenbarung ist jedoch nicht auf die zwei beispielhaften Manipulationen und die zwei beispielhaften Medikamente/Therapien beschränkt. Insgesamt kann eine Vielzahl von Manipulationen und von Medikamenten/Therapien definiert sein.

Vorzugsweise ist einer Bedienperson oder einer Bediengruppe ein Authentisierungsmittel / Authentisierungsschlüssel zugewiesen/zugeordnet.

Bevorzugt ist der Identitätskennung zumindest ein Authentisierungsmittel zugeordnet. Damit ist eine Identifizierung beim Authentisieren der Bedienperson möglich.

Bevorzugt weist ein Authentisierungsmittel zum Vorweisen einer Identität einen numerischen Code oder einen alphanumerischen Code oder eine biometrische Kennung oder eine RFID-Kennung auf. Der numerische Code und/oder der alphanumerische Code können ein durch eine Bedienperson festgelegtes Passwort sein, alternativ aber auch ein der Bedienperson zugeordnetes Merkmal, beispielsweise ein spezifischer Code, insbesondere eine Personalnummer, sein. Eine biometrische Kennung kann dabei ein Fingerabdruck, ein Gesichtsscan oder eine Iriskennung sein.

Der Datenspeicher kann als ein interner Speicher in dem medizinischen Gerät angeordnet sein oder als ein externer Speicher ausgebildet sein, welcher mit dem medizinischen Gerät verbunden ist.

Die Authentisierungsvorrichtung kann als eine interne Vorrichtung in dem medizinischen Gerät angeordnet sein oder als eine externe Vorrichtung ausgebildet sein, welche mit dem medizinischen Gerät verbunden ist.

Die Authentisierungsvorrichtung kann ein Lesegerät zum Scannen des Authentisierungsmittels aufweisen oder ein Eingabegerät zur Eingabe des Authentisierungsmittels aufweisen oder einen Datenempfänger, beispielsweise ein RFID-Empfänger oder NFC-Empfänger aufweisen. Das Eingabegerät kann als eine (externe) Tastatur oder als Bedientasten an der Authentisierungsvorrichtung ausgebildet sein oder als ein Touchscreen ausgebildet sein.

Die Authentifizierungsvorrichtung kann als eine interne Vorrichtung in dem medizinischen Gerät angeordnet sein oder als eine externe Vorrichtung ausgebildet sein, welche mit dem medizinischen Gerät verbunden ist.

Die Berechtigungsfreigabe-Auswahlvorrichtung kann als eine interne Vorrichtung in dem medizinischen Gerät angeordnet sein oder als eine externe Vorrichtung ausgebildet sein, welche mit dem medizinischen Gerät verbunden ist.

Von Vorteil ist, wenn die Authentisierungsvorrichtung und die Authentifizierungsvorrichtung mittels einer (system-internen) Datenkommunikationsleitung miteinander verbunden sind und/oder die Berechtigungsfreigabe-Auswahlvorrichtung und die Authentifizierungsvorrichtung mittels einer (system-internen) Datenkommunikationsleitung miteinander verbunden sind und/oder die Berechtigungsfreigabe-Auswahlvorrichtung und die Authentisierungsvorrichtung mittels einer (system-internen) Datenkommunikationsleitung miteinander verbunden sind. Alternativ können die Authentisierungsvorrichtung und die Authentifizierungsvorrichtung mittels einer drahtlosen Kommunikationsverbindung miteinander verbunden sein und/oder die Berechtigungsfreigabe-Auswahlvorrichtung und die Authentifizierungsvorrichtung können mittels einer drahtlosen Kommunikationsverbindung miteinander verbunden sein und/oder die Berechtigungsfreigabe-Auswahlvorrichtung und die Authentisierungsvorrichtung können mittels einer drahtlosen Kommunikationsverbindung miteinander verbunden sein.

Von Vorteil ist, wenn der Datenspeicher mit der Authentisierungsvorrichtung und/oder der Authentifizierungsvorrichtung und/oder der Berechtigungsfreigabe-Auswahlvorrichtung mittels (jeweils) einer (system-internen) Datenkommunikationsleitung oder einer drahtlosen Kommunikationsverbindung verbunden ist.

Die drahtlose Kommunikationsverbindung kann mithilfe von Wi-Fi, Bluetooth oder NFC umgesetzt sein.

Vorzugsweise kann das medizinische Gerät mit dem Datenspeicher und/oder der Authentisierungsvorrichtung und/oder der Authentifizierungsvorrichtung und/oder der Berechtigungsfreigabe-Auswahlvorrichtung mittels (jeweils) einer (system-internen) Datenkommunikationsleitung oder einer drahtlosen Kommunikationsverbindung verbunden sein. In anderen Worten kann das medizinische Gerät so ausgestaltet sein, dass es gleichzeitig als Authentisierungsvorrichtung, als Authentifizierungsvorrichtung und/oder als Berechtigungsfreigabe-Auswahlvorrichtung funktioniert. Damit ist ein besonders kompaktes Sicherungssystem gebildet.

Bei einer rein system-internen Datenkommunikationsleitung zur Kommunikation der Vorrichtungen und dem Datenspeicher untereinander bzw. miteinander sowie dem medizinischen Gerät kann das Sicherungssystem als eine Insellösung ausgebildet sein. Eine Insellösung weist vorliegend den Vorteil auf, dass das Sicherungssystem alleinstehend funktioniert. Damit ist das Sicherungssystem vor äußeren Einflüssen / Eingriffen in Form von beispielsweise Hackerangriffen geschützt.

Die Authentifizierungsvorrichtung kann eine Servereinheit sein, welche per Funk mit den anderen Vorrichtungen und dem medizinischen Gerät kommuniziert.

Die Offenbarung betrifft auch ein Verfahren zur Erteilung von identitätsspezifischen Berechtigungsfreigaben an Bedienpersonen für das Ausführen von berechtigungsabhängigen Manipulationen an einem medizinischen Gerät.

Zuerst werden in einen ersten Datenspeicher oder Datensatz Daten eingegeben, die eine Mehrzahl von Bedienpersonengruppen oder Hierarchiestufen ausweisen, denen zueinander unterschiedlichen Autorisierungsgrade zugeordnet werden, wobei jedem Autorisierungsgrad auswählbare Manipulationen am medizinischen Gerät zugewiesen werden. Weiterhin werden in einem zweiten Datenspeicher oder Datensatz Daten eingegeben, die eine Mehrzahl von unterschiedlichen Medikamenten und/oder Therapien ausweisen, denen jeweils auswählbare, freizuschaltende Manipulationen oder zumindest einer der zueinander unterschiedlichen Autorisierungsgrade gemäß der ersten Datenbank /dem ersten Datensatz zugeordnet werden. Schließlich wird einer Bedienperson in Abhängigkeit eines von dieser erbrachten Berechtigungsnachweises zumindest eine der Hierarchiestufen aus dem ersten Datenspeicher/Datensatz zugeordnet und jene darin enthaltenen Manipulationen freigeschaltet, welche mit den Medikamenten-/Therapie-bezogenen Manipulationen gemäß dem zweiten Datenspeicher/Datensatz übereinstimmen.

Vorzugsweise wird zur Erbringung des Berechtigungsnachweises zuerst ein Authentisierungsmittel an einer Authentisierungsvorrichtung zur Feststellung einer Identität der Bedienperson bereitgestellt. Anschließend werden die von der Bedienperson übermittelten Identifikationsdaten von der Authentisierungsvorrichtung erkannt. Nachfolgend werden die Daten an eine Authentifizierungsvorrichtung zur Verifikation der Daten übertragen. Dann wird die Authentifizierung zur Bestätigung der Identität anhand von in einem Datenspeicher hinterlegten Anzahl von Bedienpersonen oder Bediengruppen/Hierarchiestufen durchgeführt. Zuletzt erfolgt eine Erteilung der Berechtigungsfreigabe nach erfolgreicher Authentifizierung in Abhängigkeit der Hierarchie-/ Berechtigungsstufe der authentifizierten Bedienperson und der Medikamenten-/Therapie-bezogenen Manipulationen durch eine Berechtigungsfreigabe-Auswahlvorrichtung.

Von Vorteil ist, wenn die Berechtigungsfreigabe-Auswahlvorrichtung für die Berechtigungsfreigabe einen Abgleich der Berechtigungsstufen vom ersten Datensatz mit der, der Bedienperson zugewiesenen Berechtigungsstufe vornimmt und bei einer Übereinstimmung der Berechtigungsstufen die Berechtigungsfreigabe erteilt.

Die Offenbarung wird nachstehend anhand eines bevorzugten Ausführungsbeispiels unter Bezugnahme auf die begleitenden Figuren näher erläutert.
Fig. 1 ist eine schematische Ansicht eines Sicherungssystems in einer ersten Ausführungsform;
Fig. 2 ist eine schematische Ansicht des Sicherungssystems in einer zweiten Ausführungsform
Fig. 3 ist eine schematische Ansicht des Sicherungssystems in einer dritten Ausführungsform,
Fig. 4 ist ein Ablaufdiagramm zur Erteilung einer Berechtigungsfreigabe.

### Beschreibung der Ausführungsbeispiele

Nachstehend werden Ausführungsbeispiele der vorliegenden Offenbarung auf der Basis der zugehörigen Figuren beschrieben.

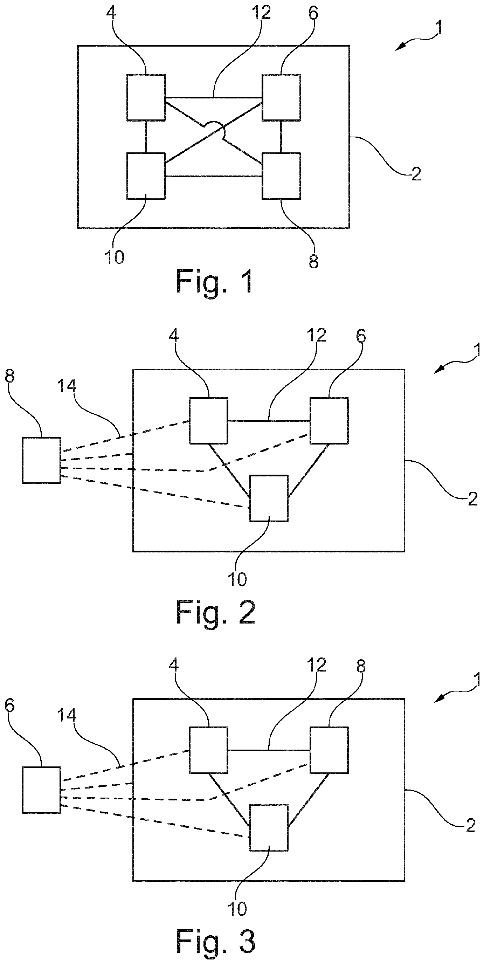

Fig. 1 zeigt eine schematische Ansicht eines elektronischen Zugangs-Sicherungssystems 1 in einer ersten Ausführungsform. Das Sicherungssystem 1 ist vorliegend für ein medizinisches Gerät, insbesondere eine Infusionspumpe 2 dargestellt. Das Sicherungssystem 1 ist zur Erteilung von identitätsspezifisch auswählbaren Berechtigungsfreigaben an Bedienpersonen für das Ausführen von Berechtigungs- und Medikamenten-/Therapie-abhängigen Manipulationen am medizinischen Gerät 2 vorgesehen.

Das Sicherungssystem 1 weist hierfür wenigstens einen Datenspeicher 4 auf. Der Datenspeicher 4 umfasst einen ersten Datensatz der Daten bezüglich einer Mehrzahl von Hierarchie-/Berechtigungsstufen enthält, denen jeweils bestimmte Manipulationen zugewiesen sind. Die Manipulationen können sich unter den jeweiligen Hierarchie-/Berechtigungsstufen zumindest teilweise unterscheiden, sich teilweise gleichen oder die Anzahl an Manipulationen können unter den jeweiligen Hierarchie-/Berechtigungsstufen voneinander abweichen. Der Datenspeicher 4 umfasst einen zweiten Datensatz der Daten bezüglich einer Mehrzahl von Medikamenten und/oder Therapien enthält, denen jeweils bestimmte freizuschaltende Manipulationen zugeordnet sind. Die freizuschaltenden Manipulationen können sich unter den jeweiligen Medikamenten/Therapien zumindest teilweise unterscheiden, sich teilweise gleichen oder die Anzahl an Manipulationen können unter den jeweiligen Medikamenten/Therapien voneinander abweichen.

Weiterhin weist das Sicherungssystem 1 eine Authentisierungsvorrichtung 6 auf. Die Authentisierungsvorrichtung 6 ist dazu vorgesehen, die Identität der aktuellen Bedienperson festzustellen.

Ferner weist das Sicherungssystem 1 eine Authentifizierungsvorrichtung 8 auf. Die Authentifizierungsvorrichtung 8 ist dazu ausgebildet, die Identität der authentisierten Bedienperson anhand der im Datenspeicher 4 abgespeicherten Daten zu bestätigen.

Das Sicherungssystem 1 weist schließlich eine Berechtigungsfreigabe- Auswahlvorrichtung 10 auf. Diese ist dafür vorgesehen und ausgebildet, bei einer erfolgreichen Authentifizierung eine (vor-)bestimmte Berechtigungsfreigabe in Abhängigkeit der Hierarchie-/Berechtigungsstufe der authentifizierten Bedienperson auszuwählen und zu erteilen. Im Rahmen dieser Berechtigungsfreigabe werden der betreffenden Bedienperson jene Manipulationen freigeschaltet, welche zum einen in der zugeordneten Hierarchie-/Berechtigungsstufe gemäß dem ersten Datensatz enthalten sind und zum anderen mit den, für das aktuelle Medikament oder der aktuellen Therapie freizuschaltenden Manipulationen gemäß dem zweiten Datensatz übereinstimmen.

In der vorliegenden ersten Ausführungsform ist das Sicherungssystem 1 als eine Insellösung ausgestaltet. Die Authentisierungsvorrichtung 6, die Authentifizierungsvorrichtung 8, der Datenspeicher 4 und die Berechtigungsfreigabe-Auswahlvorrichtung 10 sind intern in dem medizinischen Gerät 2 gebildet/angeordnet. Die Vorrichtungen 6, 8, 10 und der Datenspeicher 4 sind untereinander mithilfe von Datenübertragungsleitungen 12 miteinander verbunden/gekoppelt. Damit können Daten, die für die Erteilung der Berechtigungsfreigabe verwendet werden, untereinander ausgetauscht werden.

Fig. 2 zeigt das Sicherungssystem 1 in einer zur Fig. 1 verschiedenen Ausgestaltung, einer zweiten Ausführungsform. Vorliegend ist die Authentifizierungsvorrichtung 8 extern zu dem medizinischen Gerät 2 ausgebildet/angeordnet. Anstatt einer internen Datenübertragungsleitung 12 wie in Fig. 1 dargestellt erfolgt der Datentransfer der Authentifizierungsvorrichtung 8 über eine Funkverbindung 14. Somit ist die Authentifizierungsvorrichtung mithilfe der Funkverbindung 14 mit dem medizinischen Gerät 2 und den anderen Vorrichtungen 6, 10 und dem Datenspeicher 4 verbunden.

Fig. 3 zeigt das Sicherungssystem 1 in einer dritten Ausführungsform. Hierbei ist die Authentisierungsvorrichtung 6 als eine zum medizinischen Gerät 2 separaten Vorrichtung ausgebildet. Die Authentisierungsvorrichtung 6 ist mithilfe einer Funkverbindung 14 mit dem medizinischen Gerät 2 und den anderen Vorrichtungen 8, 10 und dem Datenspeicher 4 verbunden.

Die Fign. 1 bis 3 zeigen voneinander verschiedene beispielhafte Ausführungsformen. Das offenbarte Sicherungssystem 1 ist jedoch nicht auf diese Ausführungsformen beschränkt. Der Datenspeicher 4 und/oder die Berechtigungsfreigabe- Auswahlvorrichtung 10 können ebenfalls wie in den Fign. 1 bis 3 dargestellten Ausführungen extern zum medizinischen Gerät 2 ausgeführt sein und wahlweise eine Funkverbindung 14 oder eine Kabelverbindung aufweisen.

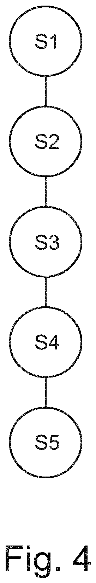

Fig. 4 zeigt ein Ablaufdiagramm zur Erteilung der Berechtigungsfreigabe.

Zuerst wird in einem ersten Schritt (S1) ein Authentisierungsmittel an der Authentisierungsvorrichtung 6 zur Feststellung einer Identität, vorliegend der Identität der Bedienperson, bereitgestellt.

In einem zweiten Schritt (S2) erkennt die Authentisierungsvorrichtung 6 das Authentisierungsmittel und ermittelt anhand der im Datenspeicher 4 hinterlegten Daten die Bedienperson.

In einem dritten Schritt (S3) werden die Daten der Identität von der Authentisierungsvorrichtung 6 an die Authentifizierungsvorrichtung 8 übertragen, um eine Authentifizierung, vorliegend eine Verifizierung der Daten, ergo der Bedienperson, durchzuführen.

In einem vierten Schritt (S4) erfolgt die Authentifizierung.

Wenn die Authentifizierung der Bedienperson erfolgreich ist, erfolgt in einem fünften Schritt (S5) mithilfe der Berechtigungsfreigabe-Auswahlvorrichtung 10 ein Abgleich einer Berechtigungsstufe der Bedienperson mit einer Berechtigungsstufe der Manipulation. Stimmen die Berechtigungsstufe der Bedienperson und die Berechtigungsstufe der Manipulation überein, so ist eine Berechtigungsfreigabe erteilt. Stimmen die Berechtigungsstufe der Bedienperson und die Berechtigungsstufe der Manipulation nicht überein, so ist keine Berechtigungsfreigabe erteilt.

### Bezugszeichenliste

- 1: Sicherungssystem
- 2: medizinisches Gerät
- 4: Datenspeicher
- 6: Authentisierungsvorrichtung
- 8: Authentifizierungsvorrichtung
- 10: Berichtigungsfreigabe-Auswahlvorrichtung
- 12: interne Datenübertragungsleitung
- 14: Funkverbindung

## Patentansprüche

1. Sicherungssystem (1) eines oder für ein medizinisches Gerät (2) zur Erteilung von Berechtigungsfreigaben an Bedienpersonen für das Ausführen von berechtigungsabhängigen Manipulationen am medizinischen Gerät (2), mit
- zumindest einem Datenspeicher (4), in dem ein erster Datensatz angelegt ist, der Daten enthält, die eine Mehrzahl von Hierarchie- oder Berechtigungsstufen ausweisen, denen jeweils bestimmte oder auswählbare Manipulationen zugeordnet oder zuordenbar sind, und in dem ein zweiter Datensatz angelegt ist, der Daten enthält, die eine Mehrzahl von Medikamenten und/oder Therapien ausweisen, denen jeweils freizuschaltende Manipulationen zugeordnet oder zuordenbar sind und
- einer Berechtigungsfreigabe-Auswahlvorrichtung (10), die dafür vorgesehen und ausgebildet ist, einer Bedienperson in Abhängigkeit ihres jeweiligen Berechtigungsnachweises zumindest eine der Hierarchie- oder Berechtigungsstufen zuzuordnen und nur jene, dieser Hierarchie- oder Berechtigungsstufe zugeordneten Manipulationen gemäß dem ersten Datensatz freizuschalten, welche mit den, einem aktuell verwendeten Medikament oder einer aktuell angewandten Therapie zugeordneten, freizuschaltenden Manipulationen gemäß dem zweiten Datensatz übereinstimmen.

2. Sicherungssystem (1) nach Anspruch 1, **gekennzeichnet durch**
eine Authentisierungsvorrichtung (6), die dazu vorgesehen und ausgebildet ist, die Identität einer aktuellen Bedienperson festzustellen und
eine Authentifizierungsvorrichtung (8), die dazu vorgesehen und ausgebildet ist, die Identität der authentisierten Bedienperson anhand der im Datenspeicher (4) abgespeicherten Daten zu bestätigen.

3. Sicherungssystem (1) nach einem der vorhergehenden Ansprüche, **dadurch gekennzeichnet, dass** das medizinische Gerät (2) eine Infusionspumpe, eine Blutbehandlungsmaschine oder dergleichen intensivmedizinische Einrichtung ist.

4. Sicherungssystem (1) nach einem der vorhergehenden Ansprüche, **gekennzeichnet, durch** ein Authentisierungsmittel, das dafür vorgesehen ist, einer Bedienperson zugeordnet zu werden.

5. Sicherungssystem (1) nach Anspruch 4, **dadurch gekennzeichnet, dass** das Authentisierungsmittel zum Vorweisen einer Identität der Bedienperson einen numerischen Code oder einen alphanumerischen Code oder eine biometrische Kennung oder eine RFID- Kennung aufweist.

6. Sicherungssystem (1) nach Anspruch 2 in Verbindung mit Anspruch 5, **dadurch gekennzeichnet, dass** die Authentisierungsvorrichtung (6) ein Lesegerät zum Scannen des Authentisierungsmittels aufweist oder ein Eingabegerät zur manuellen Eingabe des Authentisierungsmittels aufweist oder einen Datenempfänger aufweist.

7. Sicherungssystem (1) nach einem der vorhergehenden Ansprüche 2 bis 7, **dadurch gekennzeichnet, dass** die Authentisierungsvorrichtung (6) und die Authentifizierungsvorrichtung (8) mittels einer system-internen Datenkommunikationsleitung (12) miteinander verbunden sind und/oder die Berechtigungsfreigabe-Auswahlvorrichtung (10) und die Authentifizierungsvorrichtung (8) mittels einer system-internen Datenkommunikationsleitung (12) miteinander verbunden sind.

8. Verfahren zur Erteilung von identitätsspezifischen Berechtigungsfreigaben an Bedienpersonen für das Ausführen von berechtigungsabhängigen Manipulationen an einem medizinischen Gerät mit den folgenden Schritten:
- Eingeben von Daten in einen ersten Datenspeicher oder Datensatz, wobei die Daten eine Mehrzahl von Bedienpersonengruppen oder Hierarchiestufen ausweisen, denen zueinander unterschiedlichen Autorisierungsgrade zugeordnet werden, wobei jedem Autorisierungsgrad auswählbare oder ausgewählte Manipulationen am medizinischen Gerät zugewiesen werden,
- Eingeben von Daten in einen zweiten Datenspeicher oder Datensatz, wobei die Daten eine Mehrzahl von unterschiedlichen Medikamenten und/oder Therapien ausweisen, denen jeweils auswählbare oder ausgewählte, freizuschaltende Manipulationen oder zumindest einem der zueinander unterschiedlichen Autorisierungsgrade gemäß dem ersten Datenspeicher /dem ersten Datensatz zugeordnet werden und
- Zuordnen von zumindest einer der Hierarchiestufen aus dem ersten Datenspeicher/Datensatz an eine Bedienperson in Abhängigkeit eines von dieser erbrachten Berechtigungsnachweises und Freischalten jener darin enthaltenen Manipulationen, welche mit den Medikamenten-/Therapiebezogenen Manipulationen gemäß dem zweiten Datenspeicher/Datensatz übereinstimmen.
